# EUROPEAN PATENT APPLICATION

(11) **EP 4 821 456 A1**
(43) Date of publication of application: **14.10.2026**
(21) Application number: 26163112.1
(22) Date of filing: 09.03.2026
(51) Int. Cl.: A61B 34/30, B25J 9/06, B25J 9/16, A61B 34/00, A61B 34/20

(54) **SYSTEM AND COMPUTER IMPLEMENTED METHOD FOR CONTROLLING A FLEXIBLE DEVICE WHILST INSPECTING OR REPAIRING A COMPONENT**

(30) Priority: 08.04.2025 GB 202505257
(71) Applicant: Rolls-Royce plc, London N1 9FX (GB)
(72) Inventor: Wild, Samuel P, Derby, DE24 8BJ (GB); Dong, Xin, Derby, DE24 8BJ (GB)
(74) Representative: Rolls-Royce plc

(57) **Abstract**

A system (100, 200, 300, 400, 700) for controlling a flexible device (102, 202, 302, 402, 702) whilst inspecting or repairing a component (104) includes a first sensor (106, 206, 306, 406, 706) coupled to the flexible device that generates an information related to a current curvature of the first sensor and a controller (112) including memories (116) and processors (114). The processors (114) receive, from the first sensor, information related to the current curvature of the first sensor, determine a predicted pose and a current shape of the first sensor, determine a current shape of the flexible device, and adjust the current shape of the flexible device.

## Description

### FIELD

This invention relates to a system for controlling a flexible device whilst inspecting or repairing a component, and a computer implemented method for controlling the flexible device whilst inspecting or repairing the component.

### BACKGROUND

Flexible devices, such as continuum robots, have emerged as versatile systems capable of intricate maneuvers and operations in complex environments including on-wing (in-situ) inspection and repair of components such as those associated with gas turbine engines. Such flexible devices are typically tendon driven. The aforesaid snake-like devices can contort to navigate obstacle-ridden environments. However, it is difficult to establish accurate sensing and control of the flexible devices that is essential for industrial adoption. For example, due to lack of accuracy in determination and control of a shape of the flexible devices during an inspection or repair operation, reliable in-situ closed-loop control of the flexible devices is challenging, which may hinder their widespread adoption. For example, a common challenge in the flexible devices is twists and/or torsion. The twist is caused due to an unintended axial rotation caused by asymmetrical actuation of tendons, and/or routing and/or kinematics of the flexible devices. The twists can degrade an accuracy in detecting the shape of the flexible devices and may complicate control algorithms of the flexible devices, particularly when the flexible device is subjected to parasitic twists.

Conventional methods of controlling the shape of the flexible devices may be time consuming and involve complexities, may involve time consuming training, and may involve numerous trial and error cycles that may not fully capture effects of twists and/or torsion. Some conventional flexible devices address the issue of torsion partially but fail to compensate for multidirectional twists or local curvature variations in the flexible devices. Thus, there exists a need for an improved technique for addressing the abovementioned shortcomings and develop a flexible device that can be controlled as per desired requirements whilst inspecting or repairing components.

European patent application EP 4570453 A1 (prior art under A54(3) EPC) discloses a method of determining the position and shape of a flexible arm robot having at least one distributed Bragg reflector integrated. The method involves: moving the flexible arm robot through a series of movements; tracking the movements of the flexible arm robot and recording positional data of at least a section of the flexible arm robot as a ground truth and interrogating the distributed Bragg reflector during these movements; inputting the data from the interrogation of the distributed Bragg reflector and the camera into a machine learning algorithm; using the output of the machine learning algorithm to determine a calibration function for the position of the distributed Bragg reflector within the flexible arm robot; and applying the calibration function to software used to control the movement of the flexible arm robot.

### SUMMARY

In a first aspect, there is provided a system for controlling a flexible device whilst inspecting or repairing a component. The system includes a first sensor coupled to the flexible device and extending along a central axis of the flexible device. The first sensor is configured to generate an information related to a current curvature of the first sensor relative to a reference point of the flexible device. The system further includes a controller. The controller includes one or more memories and one or more processors communicably coupled with the one or more memories and the first sensor. The one or more memories are configured to store an information related to a ground truth shape of the flexible device. The one or more processors are configured to receive, from the first sensor, the information related to the current curvature of the first sensor. The one or more processors are further configured to receive a set of simulated data related to the current curvature of the first sensor. The one or more processors are further configured to retrieve, from the one or more memories, the information related to the ground truth shape of the flexible device. The one or more processors are further configured to determine a predicted pose of the first sensor based on at least the information related to the current curvature of the first sensor and/or the set of simulated data related to the current curvature of the first sensor, and the information related to the ground truth shape of the flexible device. The one or more processors are further configured to determine a current shape of the first sensor based on at least the predicted pose of the first sensor. The one or more processors are further configured to determine a current shape of the flexible device based on at least the current shape of the first sensor. The one or more processors are further configured to adjust the current shape of the flexible device so that the current shape corresponds to a desired shape of the flexible device based at least on the current shape of the flexible device.

The system described herein may allow accurate control of the shape of the flexible device whilst inspection or repair of the component. In some examples, the system may be used to determine the current shape of the flexible device using neural network algorithms to subsequently correct the current shape of the flexible device, thereby eliminating any deviations between the current shape of the flexible device and the desired shape of the flexible device. The deviations may arise due to a twist in the flexible device, a friction in the flexible device, a strain in the flexible device, and/or a temperature of the flexible device. The system may eliminate the effects of twists and/or torsion in the flexible device, which may in turn provide a robust force transmission whilst inspection or repair of the component. The system may be applicable to a wide variety of flexible devices including, but not limited to, tendon-driven continuum robots.

On execution of the neural network algorithm, the processors of the system may optimise a single layer, such as a dense layer of the neural network algorithm, thereby reducing time involved in design and execution of the neural network algorithm. Thus, users may efficiently find the right model size without exhaustively searching the hyperparameter space. The proposed system may eliminate 'trial and error' cycles, may lower complexities, and may enable rapid deployment of the flexible device. The system may use the neural network algorithm as a "master network" to train smaller models that may have a different architecture. Further, continuous online recalibration of the neural network algorithm may boost accuracy of the system in changing environments and thereby improve robustness of the flexible devices. Furthermore, the system may reduce a raw root mean square error (RMSE) as a percentage of a length of the flexible device while determining the current shape of the flexible devices. Overall, the system may allow repeatability and robustness whilst correcting the current shape of the flexible device.

The system may be applicable to flexible instruments, catheters, borescopes, and any tubular device requiring measurement or compensation of twists in real-time. Further, the system may work well for continuum robots, and/or any other flexible string-like devices that may be used in inspection and repair of components of engines, automobiles, aerospace applications, medicinal applications, or any other environments. Some examples of application of the system of the present invention may include minimally invasive surgery, search and rescue operations, and so on. Further, the system may provide shape sensing of flexible robots such as, pneumatically actuated or concentric tube robots, flexible devices, such as, an endoscope or a borescope, and so on. The system may work well for active continuum robots possessing no random passive section, or for hybrid continuum robots (active or passive). The system may operate in real-time during the repair or inspection of the component and may allow monitoring and optimizing of the repair or inspection operation being performed on the component. Further, the system may enhance a service life of the flexible device via reduction in wear and tear of the components of the flexible device such as tendons of the flexible device. Furthermore, the system may enhance a performance of the inspection or repair of the component.

In some embodiments, the first sensor includes a Fibre Bragg grating (FBG) sensor. In some embodiments, the first sensor includes a single-core FBG sensor, a multi-core FBG sensor, or a FBG sensor including multiple core fibres. The FBG sensors may be used for enhanced proprioception of the flexible device because of their small diameter, flexibility, and immunity to electromagnetic interference, which may enable accurate generation of the information related to the current curvature of the first sensor relative to the reference point of the flexible device. Further, the first sensor may provide an enriched dataset corresponding to the information related to the current curvature of the first sensor.

In some embodiments, the information related to the current curvature of the first sensor is determined along each of an X-axis, a Y-axis, and a Z-axis of the flexible device. The first sensor extending along the central axis of the flexible device may allow accurate determination of the information related to the current curvature of the first sensor along each of the X-axis, the Y-axis, and the Z-axis of the flexible device. The determination of the information related to the current curvature of the first sensor along each of the X-axis, the Y-axis, and the Z-axis of the flexible device may in turn provide for the accurate determination of the information related to the current curvature of the first sensor relative to the reference point of the flexible device.

In some embodiments, the system further includes a second sensor configured to determine the ground truth shape of the flexible device. The flexible device may be actuated within a free space, or an application environment to determine the ground truth shape and/or ground truth position of the flexible device.

In some embodiments, the second sensor includes a camera, an electromagnetic tracker, a light detection and ranging (LIDAR) sensor, a radio detection and ranging (RADAR) sensor, a time of flight sensor, and/or an inertial measurement unit (IMU). The second sensor may include one or more of the aforesaid sensors to accurately determine the ground truth shape of the flexible device. In some examples, the ground truth shape may be recorded using motion tracking cameras or Red, Green, Blue and Depth (RGB-D) cameras.

In some embodiments, the set of simulated data is generated based on at least the information related to a plurality of historical values for the current curvature of the first sensor received from the first sensor and a plurality of historical values for the ground truth shape of the flexible device received from the second sensor. The set of simulated data may be generated by using statistical or machine learning models based on at least the information related to the plurality of historical values for the current curvature of the first sensor and the plurality of historical values for the ground truth shape of the flexible device. The historical values for the current curvature of the first sensor and the historical values for the ground truth shape may be simulated within a realistic environment. In some examples, the current curvature of the first sensor may be simulated using a physics simulator, for example finite element analysis (FEA) or kinematics simulation, and the ground truth data may be collected from the model pose in the simulation.

In some embodiments, a routing of the first sensor includes a straight routing, a helical routing, a hybrid routing, a braided routing, a routing at various radii, a zig-zag routing, an asymmetric routing, and/or a segmented routing. The aforesaid helical routing of the first sensor may reduce torsion of the flexible device due to redistribution of torsion into axial and torsional loads. In some examples, the helical routing of the first sensor may also reduce torsional friction, and thus, the system may be applicable to applications where friction in a concern such as, due to longer path lengths, usage of lubricants, usage of coatings, and/or usage of low-friction channel liners. The aforesaid helical routing may also ease the determination of the information related to the current curvature of the flexible device and improve the control of the flexible device.

In some embodiments, the flexible device includes a backbone having a plurality of vertebrae and a plurality of tendons circumferentially arranged around the backbone at various angular positions. The central axis of the flexible device is defined by the backbone of the flexible device. The first sensor is disposed proximal to the backbone of the flexible device or proximal to an outer surface defined by the flexible device. Thus, the system of the present invention may be used to adjust the current shape of the flexible devices such as different types of tendon driven robots.

In some embodiments, at least one of the plurality of tendons is the first sensor or the first sensor is coupled to any one of the plurality of tendons. Specifically, if at least one of the plurality of tendons is the first sensor, the first sensor itself operates as the tendon of the flexible device and may be used to control a movement of the flexible device. The inclusion of the first sensor as the tendon or the coupling of the first sensor with the tendon may ensure precise consideration of dynamics of the tendons such as direct forces exerted by the tendons, thereby providing accurate information related to the current curvature of the first sensor.

In some embodiments, the reference point is defined at the backbone of the flexible device. The determination of the information of the current curvature of the first sensor relative to the backbone of the flexible device may simplify the determination of the information related to the current curvature of the first sensor.

In some embodiments, the one or more processors are configured to implement a neural network algorithm to determine the predicted pose of the first sensor. The data in the form of shape data of the first sensor and the ground truth shape of the flexible device is standardised to a common scale so that it is ready to train the neural network algorithm. The neural network algorithm may use machine learning techniques to accurately determine the predicted pose of the first sensor. The neural network algorithm may provide a relationship between the current curvature of the first sensor and the ground truth shape of the flexible device. In an example, the neural network algorithm may adjust a tip of the flexible device. The neural network algorithm may be provided for each position of the first sensor at multiple angular positions and at multiple sections of the flexible device to accurately predict the relationship between the current shape of the first sensor against the ground truth shape of the flexible device. The neural network algorithm may include additional modalities as inputs to increase an effectiveness of determination of the predicted pose of the first sensor. The various predicted poses of the first sensor from the neural network algorithm may also be used to train the neural network algorithm.

In some embodiments, the flexible device includes a driver circuit communicably coupled to the one or more processors. The driver circuit is configured to adjust the current shape of the flexible device so that the current shape corresponds to the desired shape of the flexible device. The driver circuit may include high-voltage amplifiers, high-voltage switching circuits such as Metal-Oxide-Semiconductor Field-Effect Transistor (MOSFET)-based or Insulated Gate Bipolar Transistor (IGBT)-based switching circuits, electrostatic drive controllers, and/or embedded control systems with high-voltage modules, without any limitations. Based on deviations of the current shape from the desired shape of the flexible device, the one or more processors may direct the driver circuit to adjust the current shape of the flexible device.

In some embodiments, the driver circuit includes an actuator and/or a servo circuit. The processors may control the actuator and/or the servo circuit based on an input command sent to the actuator and/or the servo circuit. The driver circuit may modulate an electric field as a function of the input commands. Thereby the driver circuit is adapted to move the flexible device in radial, axial, and/or inclined directions relative to the central axis of the flexible device. This adjusts the current shape of the flexible device so that the current shape corresponds to the desired shape of the flexible device.

In some embodiments, the flexible device is a continuum robot, a borescope, or a catheter. Thus, the system may be applicable to flexible devices such as the continuum robots used in industries, medical continuum robots such as endoscopes, snake-like surgical tools, the catheter, and/or the borescopes.

In some embodiments, the component is a gas turbine engine component. The system may be used for controlling the flexible device whilst inspecting or repairing various components associated with a gas turbine engine that typically have a complex geometry and are difficult to access.

In some embodiments, the current shape of the flexible device is different from the desired shape of the flexible device at least due to a twist in the flexible device, a friction in the flexible device, a strain in the flexible device, and/or a temperature of the flexible device. The accurate determination of the current shape of the flexible device during the inspection or repair of the component may be used to determine and rectify deviations of the current shape of the flexible device from the desired shape of the flexible device, which may be in turn used to control the flexible device whilst the inspection or repair of the component. The aforesaid deviations may account for at least the twist in the flexible device, the friction in the flexible device, the strain in the flexible device, and/or the temperature of the flexible device. Based upon the aforesaid deviations, the one or more processors may send commands to the driver circuit for adjusting the current shape of the flexible device.

In a second aspect, there is provided a computer implemented method for controlling a flexible device whilst inspecting or repairing a component. The computer implemented method includes a step of generating, by a first sensor coupled to the flexible device, an information related to a current curvature of the first sensor relative to a reference point of the flexible device. The first sensor extends along a central axis of the flexible device. The computer implemented method further includes a step of receiving, by one or more processors of a controller, the information related to the current curvature of the first sensor from the first sensor. The computer implemented method further includes a step of receiving, by the one or more processors, a set of simulated data related to the current curvature of the first sensor. The computer implemented method further includes a step of retrieving, from one or more memories of the controller, an information related to a ground truth shape of the flexible device. The computer implemented method further includes a step of determining, by the one or more processors, a predicted pose of the first sensor based on at least the information related to the current curvature of the first sensor and/or the set of simulated data related to the current curvature of the first sensor, and the information related to the ground truth shape of the flexible device. The computer implemented method further includes a step of determining, by the one or more processors, a current shape of the first sensor based on at least the predicted pose of the first sensor. The computer implemented method further includes a step of determining, by the one or more processors, a current shape of the flexible device based on at least the current shape of the first sensor. The computer implemented method further includes a step of adjusting, by the one or more processors, the current shape of the flexible device so that the current shape corresponds to a desired shape of the flexible device based at least on the current shape of the flexible device.

The computer implemented method described herein may allow accurate control of the shape of the flexible device whilst inspection or repair of the component. In some examples, the computer implemented method may be used to determine the current shape of the flexible device using neural network algorithms to subsequently correct the current shape of the flexible device, thereby eliminating any deviations between the current shape of the flexible device and the desired shape of the flexible device. The deviations may arise due to a twist in the flexible device, a friction in the flexible device, a strain in the flexible device, and/or a temperature of the flexible device. The computer implemented method may eliminate the effects of twists and/or torsion in the flexible device, which may in turn provide a robust force transmission whilst inspection or repair of the component. The computer implemented method may be applicable to a wide variety of flexible devices including, but not limited to, tendon-driven continuum robots.

On execution of the neural network algorithm, the processors of the computer implemented method may optimise a single layer, such as a dense layer of the neural network algorithm, thereby reducing time involved in design and execution of the neural network algorithm. Thus, users may efficiently find the right model size without exhaustively searching the hyperparameter space. The proposed computer implemented method may eliminate 'trial and error' cycles, may lower complexities, and may enable rapid deployment of the flexible device. The computer implemented method may use the neural network algorithm as a "master network" to train smaller models that may have a different architecture. Further, continuous online recalibration of the neural network algorithm may boost accuracy of the computer implemented method in changing environments and thereby improves robustness of the flexible devices. Furthermore, the computer implemented method may reduce a raw root mean square error (RMSE) as a percentage of a length of the flexible device while determining of the current shape of the flexible devices. Overall, the computer implemented method may allow repeatability and robustness whilst correcting the current shape of the flexible device.

The computer implemented method may be applicable to flexible instruments, catheters, borescopes, and any tubular device requiring measurement or compensation of twists in real-time. Further, the computer implemented method may work well for continuum robots, and/or any other flexible string-like devices that may be used in inspection and repair of components of engines, automobiles, aerospace applications, medicinal applications, or any other environments. Some examples of application of the computer implemented method of the present invention may include minimally invasive surgery, search and rescue operations, and so on. Further, the computer implemented method may provide shape sensing of flexible robots such as, pneumatically actuated or concentric tube robots, flexible devices, such as, an endoscope or a borescope, and so on. The computer implemented method may work well for active continuum robots possessing no random passive section, or for hybrid continuum robots (active or passive). The computer implemented method may operate in real-time during the repair or inspection of the component and may allow monitoring and optimizing of the repair or inspection operation being performed on the component. Further, the computer implemented method may enhance a service life of the flexible device via reduction in wear and tear of the components of the flexible device such as tendons of the flexible device. Furthermore, the computer implemented method may enhance a performance of the inspection or repair of the component.

In some embodiments, the first sensor includes a Fibre Bragg grating (FBG) sensor. The FBG sensors may be used for enhanced proprioception of the flexible device because of their small diameter, flexibility, and immunity to electromagnetic interference, which may enable accurate generation of the information related to the current curvature of the first sensor relative to the reference point of the flexible device. Further, the first sensor may provide an enriched dataset corresponding to the information related to the current curvature of the first sensor.

In some embodiments, a second sensor associated with the flexible device is configured to determine the ground truth shape of the flexible device. The second sensor includes a camera, an electromagnetic tracker, a light detection and ranging (LIDAR) sensor, a radio detection and ranging (RADAR) sensor, a time of flight sensor, and/or an inertial measurement unit. The flexible device may be actuated within a free space, or an application environment to determine the ground truth shape and/or ground truth position of the flexible device. The second sensor may include one or more of the aforesaid sensors to accurately determine the ground truth shape of the flexible device. In some examples, the ground truth shape may be recorded using motion tracking cameras or Red, Green, Blue and Depth (RGB-D) cameras.

In some embodiments, the set of simulated data is generated based on at least the information related to a plurality of historical values for the current curvature of the first sensor received from the first sensor and a plurality of historical values for the ground truth shape of the flexible device received from the second sensor. The set of simulated data may be generated by using statistical or machine learning models based on at least the information related to the plurality of historical values for the current curvature of the first sensor and the plurality of historical values for the ground truth shape of the flexible device. The historical values for the current curvature of the first sensor and the historical values for the ground truth shape may be simulated within a realistic environment. In some examples, the current curvature of the first sensor may be simulated using a physics simulator, for example finite element analysis or kinematics simulation, and the ground truth data may be collected from the model pose in the simulation.

In some embodiments, the flexible device includes a backbone having a plurality of vertebrae and a plurality of tendons circumferentially arranged around the backbone at various angular positions. The central axis of the flexible device is defined by the backbone of the flexible device. The first sensor is disposed proximal to the backbone of the flexible device or proximal to an outer surface defined by the flexible device. Thus, the computer implemented method of the present invention may be used to adjust the current shape of the flexible devices such as different types of tendon driven robots.

In some embodiments, at least one of the plurality of tendons is the first sensor or the first sensor is coupled to any one of the plurality of tendons. Specifically, if at least one of the plurality of tendons is the first sensor, the first sensor itself operates as the tendon of the flexible device and may be used to control a movement of the flexible device. The inclusion of the first sensor as the tendon or the coupling of the first sensor with the tendon may ensure precise consideration of dynamics of the tendons such as direct forces exerted by the tendons, thereby providing accurate information related to the current curvature of the first sensor.

In some embodiments, the step of determining, by the one or more processors, the predicted pose of the first sensor includes implementing, by the one or more processors, a neural network algorithm to determine the predicted pose of the first sensor. The data in the form of shape data of the first sensor and the ground truth shape of the flexible device is standardised to a common scale so that it is ready to train the neural network algorithm. The neural network algorithm may use machine learning techniques to accurately determine the predicted pose of the first sensor. The neural network algorithm may provide a relationship between the current curvature of the first sensor and the ground truth shape of the flexible device. In an example, the neural network algorithm may adjust a tip of the flexible device. The neural network algorithm may be provided for each position of the first sensor at multiple angular positions and at multiple sections of the flexible device to accurately predict the relationship between the current shape of the first sensor against the ground truth shape of the flexible device. The neural network algorithm may include additional modalities as inputs to increase an effectiveness of determination of the predicted pose of the first sensor. The various predicted poses of the first sensor from the neural network algorithm may also be used to train the neural network algorithm.

In some embodiments, the flexible device is a continuum robot, a borescope, or a catheter. Thus, the computer implemented method may be applicable to flexible devices such as the continuum robots used in industries, medical continuum robots such as endoscopes or snake-like surgical tools or the catheter, and/or the borescopes.

The details of one or more examples of the disclosure are set forth in the accompanying drawings and the description below. Other features, objects, and advantages of the disclosure will be apparent from the description and drawings, and from the claims.

The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

### BRIEF DESCRIPTION OF THE DRAWINGS

Embodiments will now be described by way of example only, with reference to the Figures, in which:
**FIG. 1** shows a sectional side view of a gas turbine engine;
**FIG. 2A** shows a schematic perspective view of a system for controlling a flexible device whilst inspecting or repairing a component, in accordance with an embodiment of the present invention;
**FIG. 2B** shows a schematic perspective view of a system for controlling a flexible device whilst inspecting or repairing the component, in accordance with another embodiment of the present invention;
**FIG. 2C** shows a schematic perspective view of a system for controlling a flexible device whilst inspecting or repairing the component, in accordance with yet another embodiment of the present invention;
**FIG. 3** shows a system for controlling a flexible device whilst inspecting or repairing the component, in accordance with another embodiment of the present invention;
**FIG. 4** shows a system for controlling a flexible device whilst inspecting or repairing the component, in accordance with another embodiment of the present invention;
**FIG. 5** shows a perspective view of the system of FIG. 4 having a second sensor, in accordance with an embodiment of the present invention;
**FIG. 6** shows a visual representation of an exemplary neural network algorithm;
**FIG. 7** shows exemplary graphs depicting variation in weights and kernel indexes for the neural network algorithm; and
**FIGS. 8A** **and** **8B** show a flowchart of a computer implemented method for controlling the flexible device whilst inspecting or repairing the component.

### DETAILED DESCRIPTION

Aspects and embodiments of the present invention will now be discussed with reference to the accompanying figures. Further aspects and embodiments will be apparent to those skilled in the art.

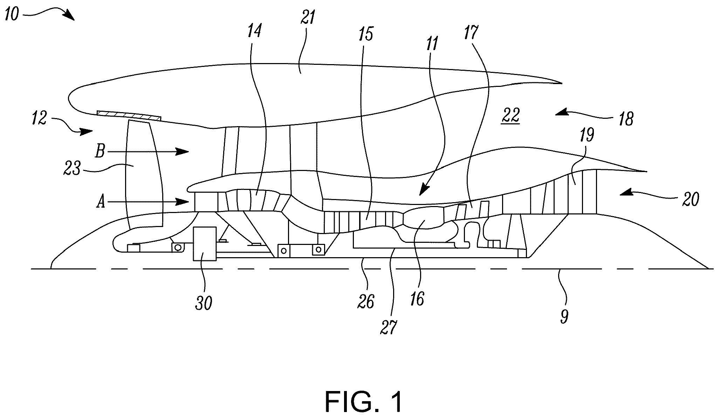

**FIG. 1** illustrates a gas turbine engine 10 having a principal rotational axis 9. The engine 10 comprises an air intake 12 and a propulsive fan 23 that generates two airflows: a core airflow A and a bypass airflow B. The gas turbine engine 10 comprises a core 11 that receives the core airflow A. The engine core 11 comprises, in axial flow series, a low pressure compressor 14, a high pressure compressor 15, combustion equipment 16, a high pressure turbine 17, a low pressure turbine 19, and a core exhaust nozzle 20. A nacelle 21 surrounds the gas turbine engine 10 and defines a bypass duct 22 and a bypass exhaust nozzle 18. The bypass airflow B flows through the bypass duct 22. The fan 23 is attached to and driven by the low pressure turbine 19 via a shaft 26 and an epicyclic gearbox 30.

In use, the core airflow A is accelerated and compressed by the low pressure compressor 14 and directed into the high pressure compressor 15 where further compression takes place. The compressed air exhausted from the high pressure compressor 15 is directed into the combustion equipment 16 where it is mixed with fuel and the mixture is combusted. The resultant hot combustion products then expand through, and thereby drive, the high pressure and low pressure turbines 17, 19 before being exhausted through the core exhaust nozzle 20 to provide some propulsive thrust. The high pressure turbine 17 drives the high pressure compressor 15 by a suitable interconnecting shaft 27. The fan 23 generally provides the majority of the propulsive thrust. The epicyclic gearbox 30 is a reduction gearbox.

Note that the terms "low pressure turbine" and "low pressure compressor" as used herein may be taken to mean the lowest pressure turbine stages and lowest pressure compressor stages (i.e., not including the fan 23) respectively and/or the turbine and compressor stages that are connected together by the interconnecting shaft 26 with the lowest rotational speed in the engine (i.e., not including the gearbox output shaft that drives the fan 23). In some literature, the "low pressure turbine" and "low pressure compressor" referred to herein may alternatively be known as the "intermediate pressure turbine" and "intermediate pressure compressor". Where such alternative nomenclature is used, the fan 23 may be referred to as a first, or lowest pressure, compression stage.

Other gas turbine engines to which the system and method of the present invention may be applied may have alternative configurations. For example, such engines may have an alternative number of compressors and/or turbines and/or an alternative number of interconnecting shafts. By way of further example, the gas turbine engine 10 shown in FIG. 1 has a split flow nozzle 18, 20 meaning that the flow through the bypass duct 22 has its own nozzle 18 that is separate to and radially outside the core exhaust nozzle 20. However, this is not limiting, and any aspect of the present disclosure may also apply to engines in which the flow through the bypass duct 22 and the flow through the core 11 are mixed, or combined, before (or upstream of) a single nozzle, which may be referred to as a mixed flow nozzle. One or both nozzles (whether mixed or split flow) may have a fixed or variable area. Whilst the described example relates to a turbofan engine, the disclosure may apply, for example, to any type of gas turbine engine, such as an open rotor (in which the fan stage is not surrounded by a nacelle) or turboprop engine, for example. In some arrangements, the gas turbine engine 10 may not comprise a gearbox 30.

The geometry of the gas turbine engine 10, and components thereof, is defined by a conventional axis system, comprising an axial direction (which is aligned with the rotational axis 9), a radial direction (in the bottom-to-top direction in FIG. 1), and a circumferential direction (perpendicular to the page in the FIG. 1 view). The axial, radial, and circumferential directions are mutually perpendicular.

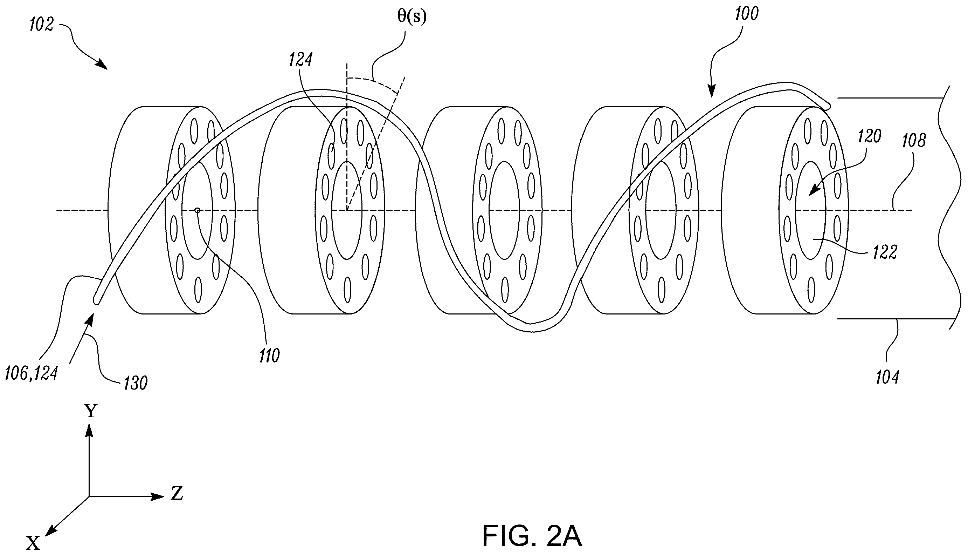

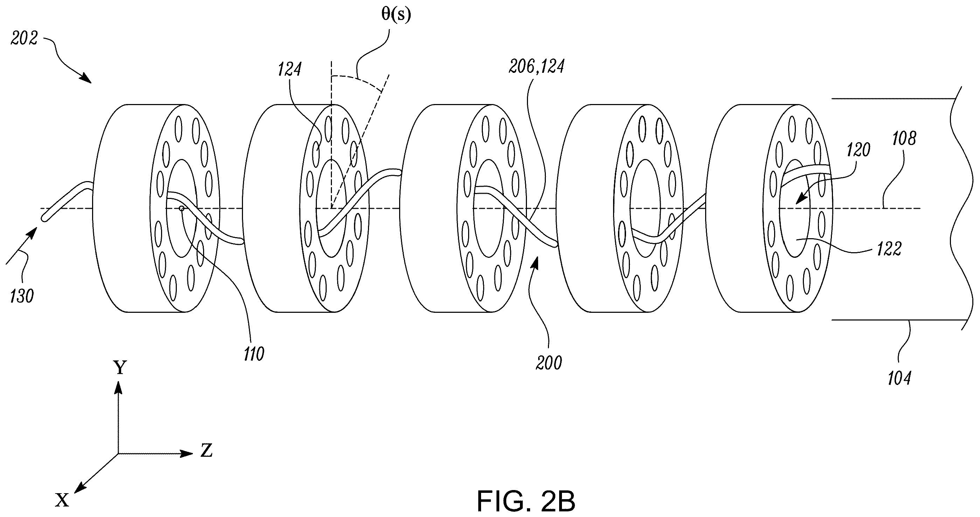

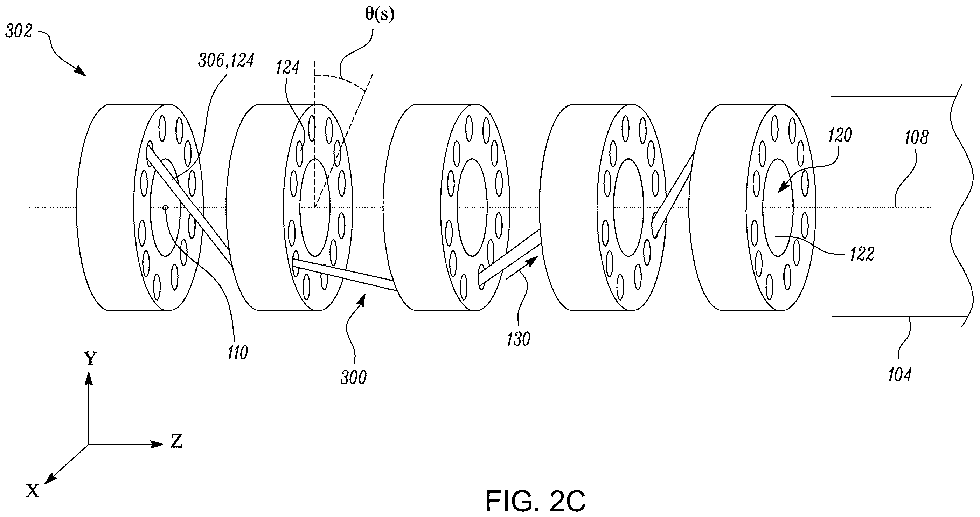

FIGS. 2A, 2B, and 2C show schematic perspective views of a flexible device 102, 202, 302,. Referring to FIGS. 2A to 2C, the present invention relates to a system 100, 200, 300 for controlling the flexible device 102, 202, 302 whilst inspecting or repairing a component 104. In an embodiment, the component 104 is a gas turbine engine 10 (shown in FIG. 1) component. The component 104 may be the turbine 17, 19 (shown in FIG. 1), the compressor 14, 15 (shown in FIG. 1), the combustion equipment 16 (shown in FIG. 1), and the like. The system 100, 200, 300 may be used for controlling the flexible device 102, 202, 302 whilst inspecting or repairing various components 104 associated with the gas turbine engine 10 that typically have complex geometry and are difficult to access. In an embodiment, the flexible device 102, 202, 302 is a continuum robot, a borescope, or a catheter. In the illustrated embodiments of FIGS. 2A to 2C, the flexible device 102, 202, 302 is the continuum robot. The system 100, 200, 300 may be applicable to flexible devices, such as continuum robots used in industries, medical continuum robots such as endoscopes, snake-like surgical tools, or the catheter, and/or the borescopes.

In an embodiment, the flexible device 102, 202, 302 includes a backbone 120 having a plurality of vertebrae 122 and a plurality of tendons 124 circumferentially arranged around the backbone 120 at various angular positions *θ*(*s*). A central axis 108 of the flexible device 102, 202, 302 is defined by the backbone 120 of the flexible device 102, 202, 302. Thus, the system 100, 200, 300 of the present invention may be used to adjust the current shape of the flexible devices 102, 202, 302 such as different types of tendon-driven robots.

Referring now to **FIG. 2A****,** the system 100 includes a first sensor 106 coupled to the flexible device 102 and extending along the central axis 108 of the flexible device 102. In the illustrated embodiment of FIG. 2A, at least one of the plurality of tendons 124 is the first sensor 106. Specifically, if at least one of the plurality of tendons 124 is the first sensor 106, the first sensor 106 itself operates as the tendon 124 of the flexible device 102 and may be used to control a movement of the flexible device 102. Such an arrangement may also ensure precise consideration of dynamics of the tendons 124 such as direct forces exerted by the tendons 124, thereby providing accurate information related to the current curvature of the first sensor 106.

The first sensor 106 is configured to generate an information related to a current curvature of the first sensor 106 relative to a reference point 110 of the flexible device 102. The information related to the curvature of the first sensor 106 may include a strain distribution at one or more locations of the first sensor 106. In some examples, the strain distributions at one or more locations of the first sensor 106 corresponding to changes in the curvature of the first sensor 106 may allow accurate generation of the information related to the current curvature of the first sensor 106.

In an embodiment, the reference point 110 is defined at the backbone 120 of the flexible device 102. The determination of the information of the current curvature of the first sensor 106 relative to the backbone 120 of the flexible device 102 may simplify the determination of the information related to the current curvature of the first sensor 106.

In an embodiment, the first sensor 106 includes a Fibre Bragg grating (FBG) sensor. In an embodiment, the first sensor 106 includes a single-core FBG sensor, a multi-core FBG sensor, or a FBG sensor including multiple core fibres. The FBG sensor includes an optical fibre. The optical fibre has at least a FBG positioned at a joint within the flexible device 102. If a bundle of fibres has been used, a FBG may be present at a point corresponding to joint positions. A Fibre Bragg grating is a length of optical fibre that has a distributed Bragg reflector constructed within a short segment of it. The Bragg reflector allows certain wavelengths to pass and others to be reflected. The optical fibres can be single, multicore or multiple core fibres. As light passes through the optical fibre, it is reflected at each of the gratings at a specific wavelength called the Bragg wavelength. The shift in the Bragg wavelengths corresponding to the strain distributions at various locations of the first sensor 106 can be used to determine the information related to the current curvature of the first sensor 106.

The FBG sensors may be used for enhanced proprioception of the flexible device 102 because of their small diameter, flexibility, and immunity to electromagnetic interference, which may enable accurate generation of the information related to the current curvature of the first sensor 106 relative to the reference point 110 of the flexible device 102. Further, the first sensor 106 may provide an enriched dataset corresponding to the information related to the current curvature of the first sensor 106.

In other examples, the first sensor 106 may include an inertial measurement unit (IMU) or any other type of sensor that provides information related to the current curvature of the first sensor 106. The present invention is not limited to a type of the first sensor 106 or a number of the first sensors 106.

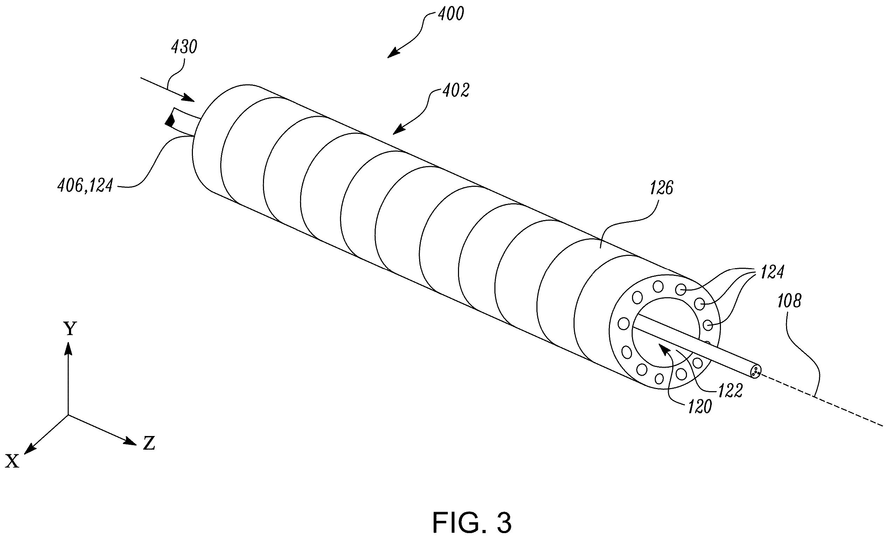

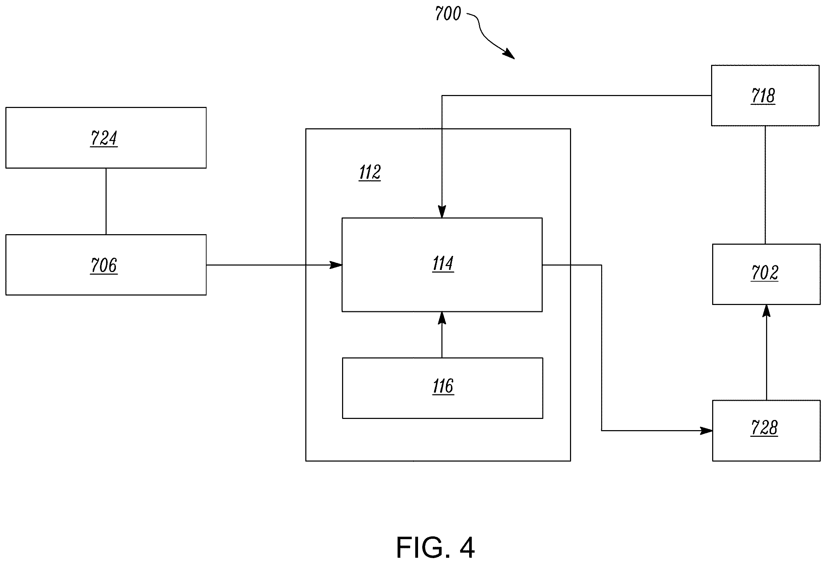

Further, the first sensor 106 is disposed proximal to the backbone 120 of the flexible device 102 and/or proximal to an outer surface 126 (shown in FIGS. 3 and 4) defined by the flexible device 102. In the illustrated embodiment of FIG. 2A, the first sensor 106 is disposed proximal to the outer surface 126 defined by the flexible device 102. It should be noted that the first sensor 106 is disposed within the flexible device 102.

In an embodiment, a routing 130 of the first sensor 106 includes a straight routing, a helical routing, a hybrid routing, a braided routing, a routing at various radii, a zig-zag routing, an asymmetric routing, and/or a segmented routing. In the illustrated embodiment of FIG. 2A, the first sensor 106 includes the helical routing 130. The aforesaid helical routing 130 of the first sensor 106 may reduce torsion of the flexible device 102 due to redistribution of torsion into axial and torsional loads. In some examples, the helical routing 130 of the first sensor 106 may also reduce torsional friction, and thus, the system 100 may be applicable to applications where friction in a concern such as, due to longer path lengths, usage of lubricants, usage of coatings, and/or usage of low-friction channel liners. The aforesaid helical routing 130 may also ease the determination of the current curvature of the flexible device 102 and improve the control of the flexible device 102.

In an embodiment, the information related to the current curvature of the first sensor 106 is determined along each of an X-axis, a Y-axis, and a Z-axis of the flexible device 102. The first sensor 106 extending along the central axis 108 of the flexible device 102 may allow accurate determination of the information related to the current curvature of the first sensor 106 along each of the X-axis, the Y-axis, and the Z-axis of the flexible device 102. The determination of the information related to the current curvature of the first sensor 106 along each of the X-axis, the Y-axis, and the Z-axis of the flexible device 102 may in turn provide for the accurate determination of the information related to the current curvature of the first sensor 106 relative to the reference point 110 of the flexible device 102.

Referring now to **FIG. 2B****,** the system 200 includes a first sensor 206. The system 200 is substantially similar to the system 100 with common components referred to by the same numeral. The first sensor 206 is substantially similar to the first sensor 106 explained in relation to FIG. 2A. However, the first sensor 206 is disposed proximal to the backbone 120 of the flexible device 202.

Referring now to **FIG. 2C****,** the system 300 includes a first sensor 306. The system 300 is substantially similar to the system 100 with common components referred to by the same numeral. The first sensor 306 is substantially similar to the first sensor 106 explained in relation to FIG. 2A. However, the first sensor 306 is disposed between the backbone 120 and the outer surface 126 defined by the flexible device 302. Specifically, some portions of the first sensor 306 is disposed proximal to the outer surface 126 and some portions of the first sensor 306 is disposed proximal to the backbone 120.

**FIG. 3** shows a perspective view of a system 400 for controlling a flexible device 402 whilst inspecting or repairing the component 104 (see FIGS. 2A to 2C), according to an embodiment of the present invention. The system 400 is substantially similar to the system 100 with common components referred to by the same numeral. The system 400 includes a first sensor 406. The first sensor 406 is substantially similar to the first sensor 106 explained in relation to FIG. 2A. The first sensor 406 is disposed proximal to the backbone 120 of the flexible device 402. Further, the first sensor 406 includes a straight routing 430 herein.

**FIG. 4** illustrates a system 700 for controlling a flexible device 702 whilst inspecting or repairing the component 104 (see FIG. 2A). The system 700 is substantially similar to the system 100 (see FIG. 2A) with common components referred to by the same numeral. The flexible device 702 is substantially similar to the flexible device 102 (see FIG. 2A) with common components referred to by the same numeral. The system 700 includes a first sensor 706. The first sensor 706 is substantially similar to the first sensor 106 explained in relation to FIG. 2A.

In the illustrated embodiment of FIG. 4, the first sensor 706 is coupled to any one of a plurality of tendons 724 of the flexible device 702. The coupling of the first sensor 706 with the tendon 724 may ensure precise consideration of dynamics of the tendons 724 such as direct forces exerted by the tendons 724, thereby providing accurate information related to the current curvature of the first sensor 706. The system 700 further includes a controller 112. The controller 112 includes one or more memories 116 and one or more processors 114 communicably coupled with the one or more memories 116 and the first sensor 706. The controller 112 may also be associated with the flexible devices 102, 202, 302, 402 (see FIGS. 2A to 2C, and FIG. 3).

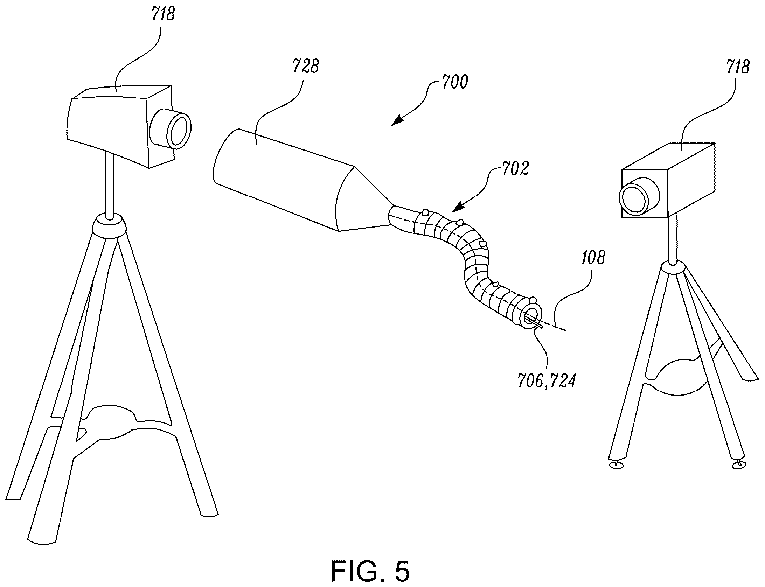

**FIG. 5** shows a perspective view of the system 700 of FIG. 4, according to an embodiment of the present invention. Referring to FIGS. 4 and 5, in an embodiment, the system 700 further includes a second sensor 718 configured to determine a ground truth shape of the flexible device 702. The flexible device 702 may be actuated within free space, or an application environment to determine the ground truth shape and/or ground truth position of the flexible device 702.

In an embodiment, the second sensor 718 includes a camera, an electromagnetic tracker, a light detection and ranging (LIDAR) sensor, a radio detection and ranging (RADAR) sensor, a time of flight sensor, and/or an IMU. The second sensor 718 may include one or more of the aforesaid sensors to accurately determine the ground truth shape of the flexible device 702. The system 700 may include a single second sensor or multiple second sensors disposed at various locations around the flexible device 702. Further, the multiple second sensors 718 may be of the same type or they may be of different types.

The camera may include motion tracking cameras and/or Red, Green, Blue, and Depth (RGB-D) cameras. The motion tracking cameras may include reflective and/or active markers (e.g., infrared light emitting diodes or retroreflective markers) that are attached to the flexible device 702. The motion tracking cameras include the markers that are positioned within the flexible device 702. The aforesaid motion tracking cameras may determine and process positions of the markers in three-dimensional space via emission and detection of infrared light from the markers and mathematical methods, respectively, to determine the ground truth shape of the flexible device 702. The RGB-D cameras calculate and process various positions on the flexible device 702 by measuring time of return of emitted pulse from the cameras and execute algorithms accordingly to determine the ground truth shape of the flexible device 702.

The electromagnetic tracker determines the position and orientation of different sections of the flexible device 702 to determine the ground truth shape of the flexible device 702. The LIDAR sensor determines time of return of emitted laser pulses from the various positions on the flexible device 702 to determine the ground truth shape of the flexible device 702. The RADAR sensor determines distance and velocity of various portions of the flexible device 702 to determine the ground truth shape of the flexible device 702. The inertial measurement unit continuously records an acceleration, an angular velocity, and/ or a magnetic field data to determine the ground truth shape of the flexible device 702. The present invention is not limited to a type of the second sensor 718.

In an embodiment, the flexible device 702 includes a driver circuit 728 communicably coupled to the one or more processors 114. The driver circuit 728 is configured to adjust a current shape of the flexible device 702 so that the current shape corresponds to a desired shape of the flexible device 702. The driver circuit 728 may include high-voltage amplifiers, high-voltage switching circuits such as Metal-Oxide-Semiconductor Field-Effect Transistor (MOSFET)-based or Insulated Gate Bipolar Transistor (IGBT)-based switching circuits, electrostatic drive controllers, and/or embedded control systems with high-voltage modules, without any limitations. Based on deviations of the current shape from the desired shape of the flexible device 702, the one or more processors 114 direct the driver circuit 728 to adjust the current shape of the flexible device 702.

In an embodiment, the driver circuit 728 includes an actuator and/or a servo circuit. The processors 114 may be configured to control the flexible device 702 based on an input command received sent to the actuator and/or the servo circuit. The driver circuit 728 modulates an electric field as a function of the input commands. Thereby the driver circuit 728 is adapted to move the flexible device 702 in radial, axial, and/or inclined directions relative to the central axis 108 (see FIGS. 2A to 2C) of the flexible device 702. This adjusts the current shape of the flexible device 702 so that the current shape corresponds to the desired shape of the flexible device 702.

Referring again to FIG. 4, the controller 112 is a device comprising hardware, software, or a combination of both along with the one or more memories 116. The controller 112 receives data and processes the data, via the processors 114, to manage the inspection or the repairing of the component 104. In some examples, the memories 116 may include a random access memory (RAM), such as synchronous dynamic random access memory (SDRAM), a read-only memory (ROM), a non-volatile random access memory (NVRAM), an electrically erasable programmable read-only memory (EEPROM), a FLASH memory, a magnetic or optical data storage media, and the like, that can be used to store various information or desired program codes in the form of instructions or data structures and that can be accessed by the processors 114.

Further, the processors 114 may execute various types of digitally stored instructions, such as software applications or algorithms, retrieved from the memories 116, or a firmware program which may enable the processors 114 to perform a wide variety of operations. It should be noted that the processors 114 may embody a single microprocessor or multiple microprocessors for receiving various input signals and generating output signals. Numerous commercially available microprocessors may perform the functions of the processors 114. Each processor 114 may further include a general processor, a central processing unit, an application specific integrated circuit (ASIC), a digital signal processor, a field programmable gate array (FPGA), a digital circuit, an analog circuit, a microcontroller, any other type of processor, or any combination thereof. Each processor 114 may include one or more components that may be operable to execute computer executable instructions or computer code that may be stored and retrieved from the memories 116.

The one or more memories 116 are configured to store an information related to the ground truth shape of the flexible device 702. The processors 114 receive the ground truth shape of the flexible device 702 from the second sensor 718, while the inspection or repair operation is being performed on the component 104 or before executing the inspection or repair operation.

The one or more processors 114 are configured to receive, from the first sensor 706, the information related to the current curvature of the first sensor 706. The processors 114 receive the information related to the current curvature of the first sensor 706, while the inspection or repair operation is being performed on the component 104.

The one or more processors 114 are further configured to receive a set of simulated data related to the current curvature of the first sensor 706. In an embodiment, the set of simulated data is generated based on at least the information related to a plurality of historical values for the current curvature of the first sensor 706 received from the first sensor 706 and a plurality of historical values for the ground truth shape of the flexible device 702 received from the second sensor 718. The set of simulated data may be generated by using statistical or machine learning models based on at least the information related to the plurality of historical values for the current curvature of the first sensor 706 and the plurality of historical values for the ground truth shape of the flexible device 702. The historical values for the current curvature of the first sensor and the historical values for the ground truth shape may be simulated within a realistic environment. In some examples, the current curvature of the first sensor may be simulated using a physics simulator, for example finite element analysis (FEA) or kinematic modelling, and the ground truth data may be collected from the model pose in the simulation.

The one or more processors 114 are further configured to retrieve, from the one or more memories 116, the information related to the ground truth shape of the flexible device 702. The processors 114 retrieve the information related to the ground truth shape of the flexible device 702, while the inspection or repair operation is being performed on the component 104.

The one or more processors 114 are further configured to determine a predicted pose of the first sensor 706 based on at least the information related to the current curvature of the first sensor 706 and/or the set of simulated data related to the current curvature of the first sensor 706, and the information related to the ground truth shape of the flexible device 702. The information related to the current curvature of the first sensor 706 and/or the set of simulated data related to the current curvature of the first sensor 706, and the information related to the ground truth shape of the flexible device 702 may provide for accurate determination of the predicted pose of the first sensor 706.

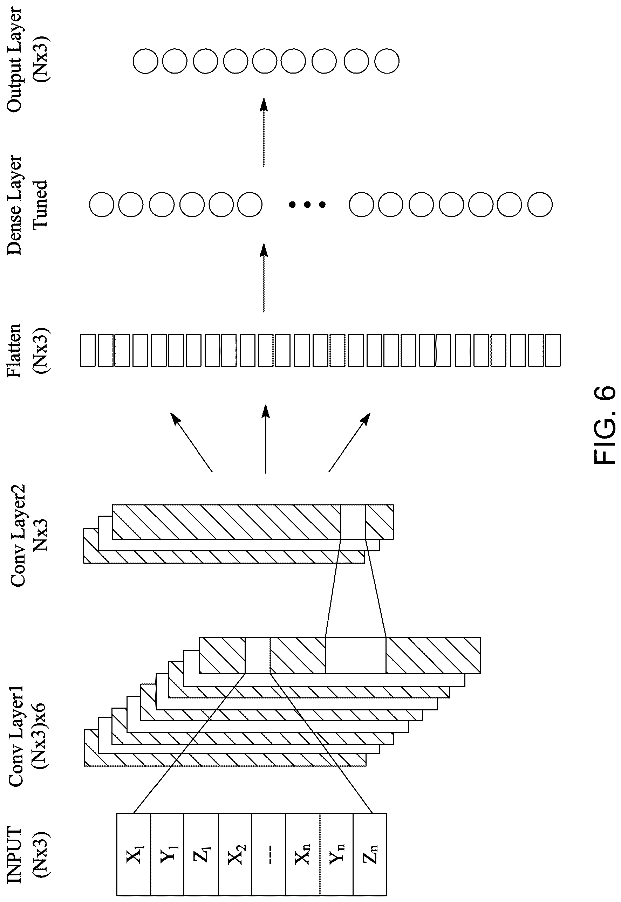

In an embodiment, the one or more processors 114 are configured to implement a neural network algorithm 502 (shown in FIG. 6) to determine the predicted pose of the first sensor 706. The data in the form of shape data of the first sensor 706 and the ground truth shape of the flexible device 702 is standardised to a common scale so that it is ready to train the neural network algorithm 502. The neural network algorithm 502 may use machine learning techniques to accurately determine the predicted pose of the first sensor 706. The neural network algorithm 502 may provide a relationship between the current curvature of the first sensor 706 and the ground truth shape of the flexible device 702. In an example, the neural network algorithm may adjust a tip of the flexible device 702. The neural network algorithm 502 may be provided for each position of the first sensor 706 at multiple angular positions *θ*(*s*) (see FIGS. 2A to 2C) and at multiple sections of the flexible device 702 to accurately predict the relationship between the current shape of the first sensor against the ground truth shape of the flexible device 702. The neural network algorithm 502 may include additional modalities as inputs to increase an effectiveness of determination of the predicted pose of the first sensor 706. The various predicted poses of the first sensor 706 from the neural network algorithm 502 may also be used to train the neural network algorithm 502.

The one or more processors 114 are further configured to determine a current shape of the first sensor 706 based on at least the predicted pose of the first sensor 706. The determination of the predicted pose of the first sensor 706 is a critical parameter and provides for accurate determination of the current shape of the first sensor 706. The one or more processors 114 may use mathematical methods and kinematic modelling to determine the current shape of the first sensor 706.

The one or more processors 114 are further configured to determine the current shape of the flexible device 702 based on at least the current shape of the first sensor 706. The processors 114 process at least the current shape of the first sensor 706 via for example, a Piecewise Constant Curvature (PCC) Model. This may allow accurate determination of the current shape of the flexible device 702.

The accurate determination of the current shape of the flexible device 702 during the inspection or repair of the component 104 may be used to determine and rectify deviations of the current shape of the flexible device 702 from the desired shape of the flexible device 702, which may be in turn used to control the flexible device 702 whilst the inspection or repair of the component 104.

The one or more processors 114 are further configured to adjust the current shape of the flexible device 702 so that the current shape corresponds to the desired shape of the flexible device 702 based at least on the current shape of the flexible device 702. Specifically, the processors 114 transmit input commands to the driver circuit 728 to control the current shape of the flexible device 702. This would remove a need for an inverse kinematics step and may allow integration of shape sensing and shape control in the same pipeline.

In an embodiment, the current shape of the flexible device 702 is different from the desired shape of the flexible device 702 at least due to a twist in the flexible device 702, a friction in the flexible device 702, a strain in the flexible device 702, and/or a temperature of the flexible device 702. Thus, the system 700 may determine and rectify deviations due to the twist in the flexible device 702, the friction in the flexible device 702, the strain in the flexible device 702, and/or the temperature of the flexible device 702.

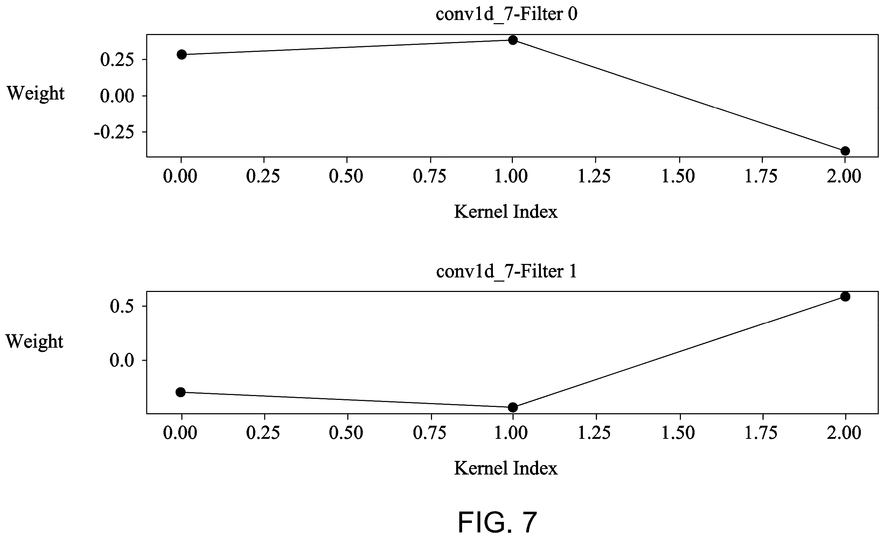

**FIG. 6** shows a visual representation of the neural network algorithm 502. **FIG. 7** shows exemplary graphs 504, 506 depicting variation in weights and kernel indexes for the neural network algorithm 502. An exemplary architecture design of the neural network algorithm 502 will now be explained in relation to FIGS. 6 and 7. A structure of the neural network algorithm 502 is general such that it can be configured for any number of positions of the first sensor 706 that are required to be considered and corrected. For example, if N is the number of the positions, the design is as follows: Layer 1) 1D Convolutional Layer (Input Layer): Number of units: N ×3, Activation Function: ReLU, Kernel Size: 6; Layer 2) 1D Convolutional Layer: Number of units: N, Activation Function: ReLU, Kernel Size: 3; Layer 3) Dense Layer: Number of units: Tuned per application, Activation Function: ReLU; Layer 4) Dense Layer, Number of units: N ×3, Activation Function: Linear.

### Description of process

The information related to the current curvature of the first sensor 706 and/or the set of simulated data related to the current curvature of the first sensor 706 are organised in a 1D sequence as an input data/modalities to the neural network algorithm 502 and the first two convolutional layers extract localised patterns such as edges, bends, and variations in shape, from the organized information and data. The convolutional layers may apply filters (kernels) to the input data to extract localised patterns. The final two fully connected layers (dense layers), the first dense layer (Layer 3) with a tuneable number of parameters as per the application and the second dense layer (Layer 4) with defined parameters, use the extracted convolutional features such as the localised patterns, to determine the predicted pose of the first sensor 706 (X_pred, Y_pred and Z_pred). For example, if the user(s) wants to track 3 positions along the flexible device 702, the neural network algorithm 502 would take the following form as shown in Table 1 below.

**TABLE 1**

| | Layer 1 | Layer 2 | Layer 3 | Layer 4 |
|---|---|---|---|---|
| Units | 9 | 3 | Tuned | 9 |
| Size of Kernel | 6 | 3 | n/a | n/a |

The size of the kernel for each of the layers may remain fixed, however, if only one point is considered the kernel for the first layer would equal 3. The tuning of the third layer (i.e., the first dense layer ) of the neural network algorithm 502 per application may be 'by hand' using trial and error or experienced judgements, and/or may be via probabilistic methods such as Bayesian optimisation to converge on suitable values in a given search space and/or via randomised selections of number of parameters for an optimal design.

The aforesaid design of the neural network algorithm 502 was tested on two prototypes of the flexible devices (similar to the flexible device 702), a short, fully actuated 3-section flexible device, and a long hybrid flexible device (passive section and active section), each with length 165 mm and 1.5 m, respectively. These two prototypes of the flexible devices 702 had two unique multicore FBGs integrated into them. Without the neural network algorithm 502, the raw root mean square error (RMSE) as a percent of a length of the flexible device 702 was 8.23% and 4.41 % and was reduced to 0.02% and 0.01%, respectively, after applying the neural network algorithm 502. This neural network algorithm 502 may learn compensation for the parasitic twist and passive section twist.

The time spent in the design of the neural network algorithm 502 is significantly reduced since only a single layer is optimised as per the flexible device 702 or application and the architecture of the three layers is known. The aforesaid design lets users efficiently find right model size without exhaustively searching the hyperparameter space. The proposed design eliminates 'trial and error' cycles, lowers technical barriers, and enables rapid deployment.

The design is modular across various flexible devices 702 and different configuration (various grating spacing, length, core count and protective sheathing) of the first sensor 706. The complexity of the design is significantly reduced as no local models for each of the first sensor 706 is required. It ensures uniform coverage across the entire flexible devices 702 and consistent performance across a base to the tip of the flexible device 702. Due to model size, inference rates in real-time are at a high frequency, achieving over 100 Hz on standard graphic processor units (GPUs), thereby reducing the barrier for the closed loop control in the flexible devices 702.

Further, the neural network algorithm 502 does not have to cover an entire length of the flexible device 702, and it could cover only a partial section of the flexible device 702, for example, the neural network algorithm 502 may cover only a region of high interest. Moreover, the present disclosure presents a single sensor (i.e., the first sensor 706) per flexible device 702, it may be contemplated that the flexible device 702 may include multiple first sensors.

Although, the neural network algorithm 502 presented herein corrects the current shape of the flexible device 702, the neural network algorithm 502 may be modified to output a three-dimensional position of a tool (not shown) attached to a position along the flexible device 702. One common example of this is an industrial or medical distal tool placed at a tip of the flexible device 702. In some examples, the present invention may cover any approach that uses any artificial augments to enhance the robustness of the neural network algorithm 502. For example, the system may receive synthetic data for training using Generative Adversarial Networks (GANs).

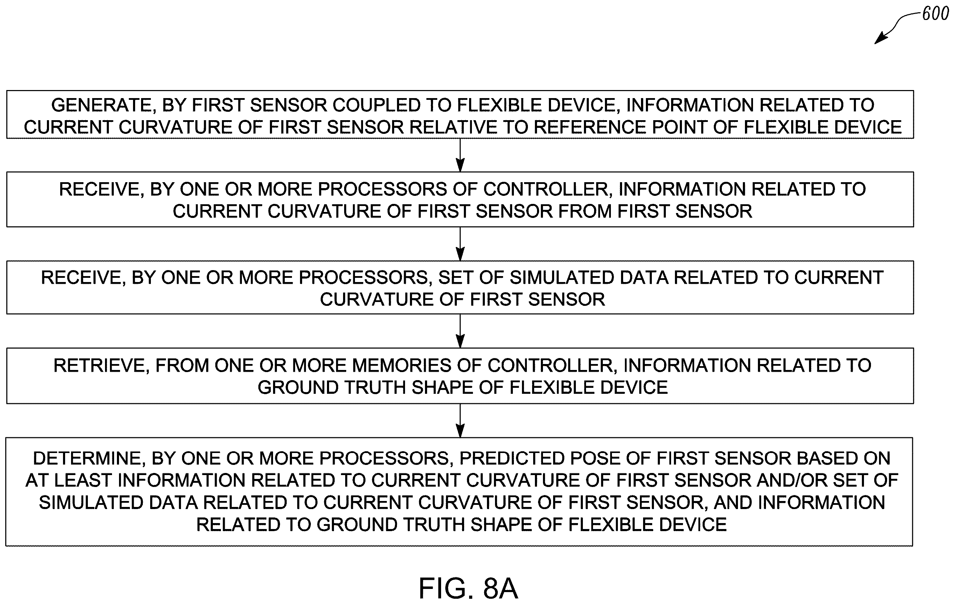

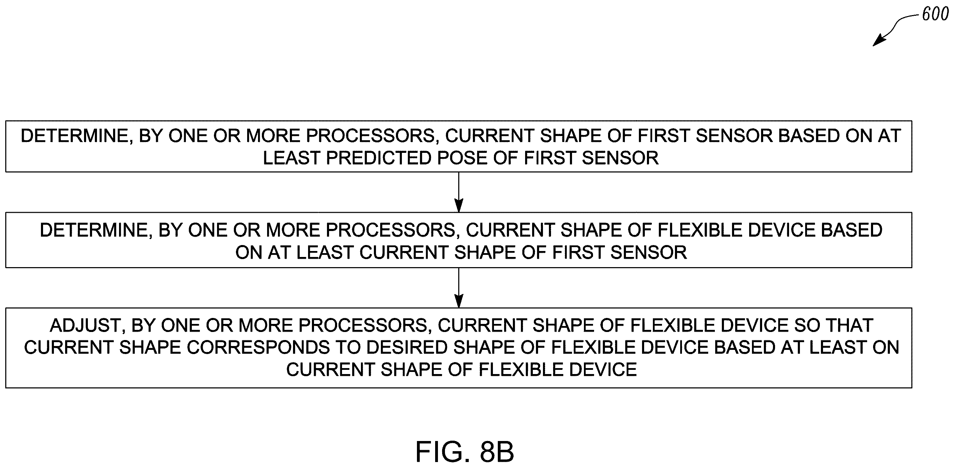

**FIGS. 8A** **and** **8B** show a flowchart of a computer implemented method 600 for controlling the flexible device 102, 202, 302, 402, 702 whilst inspecting or repairing the component 104. The computer implemented method 600 will be described with further reference to FIGS. 1 to 8B.

Referring to FIGS. 1 to 8B, the computer implemented method 600 includes a step 602 of generating, by the first sensor 106, 206, 306, 406, 706 coupled to the flexible device 102, 202, 302, 402, 702, the information related to the current curvature of the first sensor 106, 206, 306, 406, 706 relative to the reference point 110 of the flexible device 102, 202, 302, 402, 702. The first sensor 106, 206, 306, 406, 706 extends along the central axis 108 of the flexible device 102, 202, 302, 402, 702. In an embodiment, the first sensor 106, 206, 306, 406, 706 includes the FBG sensor.

In an embodiment, the flexible device 102, 202, 302, 402, 702 includes the backbone 120 having the plurality of vertebrae 122 and the plurality of tendons 124, 724 circumferentially arranged around the backbone 120 at various angular positions *θ*(*s*). The central axis 108 of the flexible device 102, 202, 302, 402, 702 is defined by the backbone 120 of the flexible device 102, 202, 302, 402, 702. The first sensor 106, 206, 306, 406, 706 is disposed proximal to the backbone 120 of the flexible device 102, 202, 302, 402, 702 or proximal to the outer surface 126 defined by the flexible device 102, 202, 302, 402, 702. In an embodiment, at least one of the plurality of tendons 124 is the first sensor 106, 206, 306, 406 or the first sensor 706 is coupled to any one of the plurality of tendons 724. In an embodiment, the flexible device 102, 202, 302, 402, 702 is the continuum robot, the borescope, or the catheter.

The computer implemented method 600 further includes a step 604 of receiving, by the one or more processors 114 of the controller 112, the information related to the current curvature of the first sensor 106, 206, 306, 406, 706 from the first sensor 106, 206, 306, 406, 706.

The computer implemented method 600 further includes a step 606 of receiving, by the one or more processors 114, the set of simulated data related to the current curvature of the first sensor 106, 206, 306, 406, 706. In an embodiment, the set of simulated data is generated based on at least the information related to the plurality of historical values for the current curvature of the first sensor 106, 206, 306, 406, 706 received from the first sensor 106, 206, 306, 406, 706 and the plurality of historical values for the ground truth shape of the flexible device 102, 202, 302, 402, 702 received from the second sensor 718.

The computer implemented method 600 further includes a step 608 of retrieving, from the one or more memories 116 of the controller 112, the information related to the ground truth shape of the flexible device 102, 202, 302, 402, 702. In an embodiment, the second sensor 718 associated with the flexible device 102, 202, 302, 402, 702 is configured to determine the ground truth shape of the flexible device 102, 202, 302, 402, 702. The second sensor 718 includes the camera, the electromagnetic tracker, the LIDAR sensor, the RADAR sensor, a time of flight sensor, and/or the IMU.

The computer implemented method 600 further includes a step 610 of determining, by the one or more processors 114, the predicted pose of the first sensor 106, 206, 306, 406, 706 based on at least the information related to the current curvature of the first sensor 106, 206, 306, 406, 706 and/or the set of simulated data related to the current curvature of the first sensor 106, 206, 306, 406, 706, and the information related to the ground truth shape of the flexible device 102, 202, 302, 402, 702. In an embodiment, the step 610 further includes implementing, by the one or more processors 114, the neural network algorithm 502 to determine the predicted pose of the first sensor 106, 206, 306, 406, 706.

The computer implemented method 600 further includes a step 612 of determining, by the one or more processors 114, the current shape of the first sensor 106, 206, 306, 406, 706 based on at least the predicted pose of the first sensor 106, 206, 306, 406, 706.

The computer implemented method 600 further includes a step 614 of determining, by the one or more processors 114, the current shape of the flexible device 102, 202, 302, 402, 702 based on at least the current shape of the first sensor 106, 206, 306, 406, 706.

The computer implemented method 600 further includes a step 616 of adjusting, by the one or more processors 114, the current shape of the flexible device 102, 202, 302, 402, 702 so that the current shape corresponds to the desired shape of the flexible device 102, 202, 302, 402, 702 based at least on the current shape of the flexible device 102, 202, 302, 402, 702.

The system 100, 200, 300, 400, 700 and the computer implemented method 600 described herein may allow accurate control of the shape of the flexible device 102, 202, 302, 402, 702 whilst inspection or repair of the component 104. In some examples, the system 100, 200, 300, 400, 700 and the computer implemented method 600 may be used to determine the current shape of the flexible device 102, 202, 302, 402, 702 using the neural network algorithm 502 to subsequently correct the current shape of the flexible device 102, 202, 302, 402, 702, thereby eliminating any deviations between the current shape of the flexible device 102, 202, 302, 402, 702 and the desired shape of the flexible device 102, 202, 302, 402, 702. The deviations may arise due to the twist in the flexible device 102, 202, 302, 402, 702, the friction in the flexible device 102, 202, 302, 402, 702, the strain in the flexible device 102, 202, 302, 402, 702, and/or the temperature of the flexible device 102, 202, 302, 402, 702. The system 100, 200, 300, 400, 700 and the computer implemented method 600 may eliminate the effects of twists and/or torsion in the flexible device 102, 202, 302, 402, 702, which may in turn provide a robust force transmission whilst inspection or repair of the component 104. The system 100, 200, 300, 400, 700 and the computer implemented method 600 may be applicable to a wide variety of flexible devices 102, 202, 302, 402, 702 including, but not limited to, tendon-driven continuum robots.

On execution of the neural network algorithm 502, the processors 114 of the system 100, 200, 300, 400, 700 and the computer implemented method 600 may optimise a single layer, such as the dense layer of the neural network algorithm 502, thereby reducing time involved in design and execution of the neural network algorithm 502. Thus, users may efficiently find the right model size without exhaustively searching the hyperparameter space. The proposed system 100, 200, 300, 400, 700 and the computer implemented method 600 may eliminate 'trial and error' cycles, may lower complexities, and may enable rapid deployment of the flexible device 102, 202, 302, 402, 702. The system 100, 200, 300, 400, 700 and the computer implemented method 600 may use the neural network algorithm 502 as a "master network" to train smaller models that may have a different architecture. Further, continuous online recalibration of the neural network algorithm 502 may boost accuracy of the system 100, 200, 300, 400, 700 and the computer implemented method 600 in changing environments and may thereby improve robustness of the flexible devices 102, 202, 302, 402, 702. Furthermore, the system 100, 200, 300, 400, 700 and the computer implemented method 600 may reduce a raw root mean square error (RMSE) as a percentage of the length of the flexible device 102, 202, 302, 402, 702 while determining of the current shape of the flexible devices 102, 202, 302, 402, 702. Overall, the system 100, 200, 300, 400, 700 and the computer implemented method 600 may allow repeatability and robustness whilst correcting the current shape of the flexible device 102, 202, 302, 402, 702.

The system 100, 200, 300, 400, 700 and the computer implemented method 600 may be applicable to flexible instruments, catheters, borescopes, and any tubular device requiring measurement or compensation of twists in real-time. Further, the system 100, 200, 300, 400, 700 and the computer implemented method 600 may work well for continuum robots, and/or any other flexible string-like devices that may be used in inspection and repair of components of engines, automobiles, aerospace applications, medicinal applications, or any other environments. Some examples of application of the system 100, 200, 300, 400, 700 and the computer implemented method 600 of the present invention may include minimally invasive surgery, search and rescue operations, and so on.

Further, the system 100, 200, 300, 400, 700 and the computer implemented method 600 may provide shape sensing of flexible robots such as, pneumatically actuated or concentric tube robots, flexible devices, such as, an endoscope or the borescope, and so on. The system 100, 200, 300, 400, 700 and the computer implemented method 600 may work well for active continuum robots possessing no random passive section, or for hybrid continuum robots (active or passive). The system 100, 200, 300, 400, 700 and the computer implemented method 600 may operate in real-time during the repair or inspection of the component 104 and may allow monitoring and optimizing of the repair or inspection operation being performed on the component 104. Further, the system 100, 200, 300, 400, 700 and the computer implemented method 600 may enhance a service life of the flexible device 102, 202, 302, 402, 702 via reduction in wear and tear of the components of the flexible device 102, 202, 302, 402, 702 such as tendons 124, 724 of the flexible device 102, 202, 302, 402, 702. Furthermore, the system 100, 200, 300, 400, 700 and the computer implemented method 600 may enhance a performance of the inspection or repair of the component 104.

It will be understood that the invention is not limited to the embodiments above-described and various modifications and improvements can be made without departing from the concepts described herein. Except where mutually exclusive, any of the features may be employed separately or in combination with any other features and the disclosure extends to and includes all combinations and sub-combinations of one or more features described herein.

## Claims

1. A system (100, 200, 300, 400, 700) for controlling a flexible device (102, 202, 302, 402, 702) whilst inspecting or repairing a component (104), the system (100, 200, 300, 400, 700) comprising:
a first sensor (106, 206, 306, 406, 706) coupled to the flexible device (102, 202, 302, 402, 702) and extending along a central axis (108) of the flexible device (102, 202, 302, 402, 702), the first sensor (106, 206, 306, 406, 706) being configured to generate an information related to a current curvature of the first sensor (106, 206, 306, 406, 706) relative to a reference point (110) of the flexible device (102, 202, 302, 402, 702); and
a controller (112) including one or more memories (116) and one or more processors (114) communicably coupled with the one or more memories (116) and the first sensor (106, 206, 306, 406, 706), the one or more memories (116) being configured to store an information related to a ground truth shape of the flexible device (102, 202, 302, 402, 702), wherein the one or more processors (114) are configured to:
receive, from the first sensor (106, 206, 306, 406, 706), the information related to the current curvature of the first sensor (106, 206, 306, 406, 706);
receive a set of simulated data related to the current curvature of the first sensor (106, 206, 306, 406, 706);
retrieve, from the one or more memories (116), the information related to the ground truth shape of the flexible device (102, 202, 302, 402, 702);
determine a predicted pose of the first sensor (106, 206, 306, 406, 706) based on at least the information related to the current curvature of the first sensor (106, 206, 306, 406, 706) and/or the set of simulated data related to the current curvature of the first sensor (106, 206, 306, 406, 706), and the information related to the ground truth shape of the flexible device (102, 202, 302, 402, 702);
determine a current shape of the first sensor (106, 206, 306, 406, 706) based on at least the predicted pose of the first sensor (106, 206, 306, 406, 706);
determine a current shape of the flexible device (102, 202, 302, 402, 702) based on at least the current shape of the first sensor (106, 206, 306, 406, 706); and
adjust the current shape of the flexible device (102, 202, 302, 402, 702) so that the current shape corresponds to a desired shape of the flexible device (102, 202, 302, 402, 702) based at least on the current shape of the flexible device (102, 202, 302, 402, 702).

2. The system (100, 200, 300, 400, 700) of claim 1, wherein the first sensor (106, 206, 306, 406, 706) includes a Fibre Bragg grating (FBG) sensor.

3. The system (100, 200, 300, 400, 700) of claim 2, wherein the first sensor (106, 206, 306, 406, 706) includes a single-core FBG sensor, a multi-core FBG sensor, or a FBG sensor including multiple core fibres.

4. The system (100, 200, 300, 400, 700) of any preceding claim, wherein the curvature of the first sensor (106, 206, 306, 406, 706) is determined along each of an X-axis, a Y-axis, and a Z-axis of the flexible device (102, 202, 302, 402, 702).

5. The system (100, 200, 300, 400, 700) of any preceding claim, further comprising a second sensor (718) configured to determine the ground truth shape of the flexible device (102, 202, 302, 402, 702), the second sensor (718) optionally includes a camera, an electromagnetic tracker, a light detection and ranging (LIDAR) sensor, a radio detection and ranging (RADAR) sensor, a time of flight sensor, and/or an inertial measurement unit.

6. The system (100, 200, 300, 400, 700) of any preceding claim, wherein the set of simulated data is generated based on at least the information related to a plurality of historical values for the current curvature of the first sensor (106, 206, 306, 406, 706) received from the first sensor and a plurality of historical values for the ground truth shape of the flexible device (102, 202, 302, 402, 702) received from the second sensor (718).

7. The system (100, 200, 300, 400, 700) of any preceding claim, wherein the flexible device (102, 202, 302, 402, 702) includes a backbone (120) having a plurality of vertebrae (122) and a plurality of tendons (124, 724) circumferentially arranged around the backbone (120) at various angular positions (*θ*(*s*)), the central axis (108) of the flexible device (102, 202, 302, 402, 702) is defined by the backbone (120) of the flexible device (102, 202, 302, 402, 702), and the first sensor (106, 206, 306, 406, 706) being disposed proximal to the backbone (120) of the flexible device (102, 202, 302, 402, 702) or proximal to an outer surface (126) defined by the flexible device (102, 202, 302, 402, 702).

8. The system (100, 200, 300, 400, 700) of claim 9, wherein: at least one of the plurality of tendons (124) is the first sensor (106, 206, 306, 406); or the first sensor (706) is coupled to any one of the plurality of tendons (724).

9. The system (100, 200, 300, 400, 700) of any preceding claim, wherein the reference point (110) is defined at the backbone (120) of the flexible device (102, 202, 302, 402, 702).

10. The system (100, 200, 300, 400, 700) of any preceding claim, wherein the one or more processors (114) are configured to implement a neural network algorithm (502) to determine the predicted pose of the first sensor (106, 206, 306, 406, 706).

11. The system (100, 200, 300, 400, 700) of any preceding claim, wherein the flexible device (102, 202, 302, 402, 702) includes a driver circuit (728) communicably coupled to the one or more processors (114), the driver circuit (728) being configured to adjust the current shape of the flexible device (102, 202, 302, 402, 702) so that the current shape corresponds to the desired shape of the flexible device (102, 202, 302, 402, 702), the driver circuit (728) optionally including an actuator and/or a servo circuit.

12. The system (100, 200, 300, 400, 700) of any preceding claim, wherein the flexible device (102, 202, 302, 402, 702) is a continuum robot, a borescope, or a catheter.

13. The system (100, 200, 300, 400, 700) of any preceding claim, wherein the component (104) is a gas turbine engine (10) component.

14. The system (100, 200, 300, 400, 700) of any preceding claim, wherein the current shape of the flexible device (102, 202, 302, 402, 702) is different from the desired shape of the flexible device at least due to a twist in the flexible device, a friction in the flexible device, a strain in the flexible device, and/or a temperature of the flexible device.

15. A computer implemented method (600) for controlling a flexible device (102, 202, 302, 402, 702) whilst inspecting or repairing a component (104), the computer implemented method (600) comprising the steps of:
generating, by a first sensor (106, 206, 306, 406, 706) coupled to the flexible device (102, 202, 302, 402, 702), an information related to a current curvature of the first sensor (106, 206, 306, 406, 706) relative to a reference point (110) of the flexible device (102, 202, 302, 402, 702), the first sensor (106, 206, 306, 406, 706) extending along a central axis (108) of the flexible device (102, 202, 302, 402, 702);
receiving, by one or more processors (114) of a controller (112), the information related to the current curvature of the first sensor (106, 206, 306, 406, 706) from the first sensor (106, 206, 306, 406, 706);
receiving, by the one or more processors (114), a set of simulated data related to the current curvature of the first sensor (106, 206, 306, 406, 706);
retrieving, from one or more memories (116) of the controller (112), an information related to a ground truth shape of the flexible device (102, 202, 302, 402, 702);
determining, by the one or more processors (114), a predicted pose of the first sensor (106, 206, 306, 406, 706) based on at least the information related to the current curvature of the first sensor (106, 206, 306, 406, 706) and/or the set of simulated data related to the current curvature of the first sensor (106, 206, 306, 406, 706), and the information related to the ground truth shape of the flexible device (102, 202, 302, 402, 702);
determining, by the one or more processors (114), a current shape of the first sensor (106, 206, 306, 406, 706) based on at least the predicted pose of the first sensor (106, 206, 306, 406, 706);
determining, by the one or more processors (114), a current shape of the flexible device (102, 202, 302, 402, 702) based on at least the current shape of the first sensor (106, 206, 306, 406, 706); and
adjusting, by the one or more processors (114), the current shape of the flexible device (102, 202, 302, 402, 702) so that the current shape corresponds to a desired shape of the flexible device (102, 202, 302, 402, 702) based at least on the current shape of the flexible device (102, 202, 302, 402, 702).
